# EUROPEAN PATENT SPECIFICATION

(11) **EP 0 359 447 B1**
(45) Date of publication and mention of the grant of the patent: **13.12.1995**
(21) Application number: 89308886.4
(22) Date of filing: 01.09.1989
(51) Int. Cl.: A61B 17/22, A61M 29/02

(54) **Improved atherectomy catheter**
Arteriektomie-Katheter
Cathéter d'athérectomie

(30) Priority: 12.09.1988 US 243397
(43) Date of publication of application: 21.03.1990
(73) Proprietor: DEVICES FOR VASCULAR INTERVENTION, INC., Redwood City, CA 94063 (US)
(72) Inventor: Gifford, Hanson S., III, Palo Alto California 94303 (US)
(74) Representative: Harrison, David Christopher

(56) References cited:
- EP-A- 163 502
- EP-A- 0 213 750
- EP-A- 0 299 158
- EP-A- 0 311 427
- DE-A- 2 740 063
- US-A- 4 323 071
- US-A- 4 669 469

## Description

The present invention relates generally to the construction of atherectomy catheters for removing atheromas from patient's arteries. In particular, the present invention relates to atherectomy devices using an inflatable balloon for effecting contact between a cutting blade and the atheroma.

Atherosclerosis is a condition characterized by fatty deposits (atheromas) in the intimal lining of a patient's arteries. Atherosclerosis can have many manifestations, including angina, myocardial infarction, strokes, hypertension, and the like. Initially, the atheromas deposited in the blood vessels remain relatively soft and tractable. Over time, however, the atheromas become calcified and hardened.

Atherectomy is a procedure which has been developed for removing atheromas from the vascular system, usually before substantial calcification has occurred. Atherectomy procedures utilize special catheters having a severing instrument located at a distal end thereof and, usually, an inflatable balloon located opposite the severing instrument. The catheter is positioned within the vascular system so that the severing instrument lies adjacent the atheroma, and the balloon is inflated to bring the severing instrument into close proximity with the atheroma. The severing instrument is then actuated to excise the atheroma, and the severed material captured to prevent the release of emboli.

The severing instrument on the atherectomy catheter can take a variety of forms, including fixed blades, (requiring movement of the entire catheter to effect cutting) and movable blades which can be actuated without movement of the catheter as a whole. Of particular interest to the present invention are atherectomy catheters having rotatable cutting elements which can be advanced across an aperture formed in a housing. The balloon is mounted on the housing so that the atheroma may be urged into the aperture with the cutting blade being advanced to sever the atheroma.

Heretofore, atherectomy devices have suffered from deficiencies in the manner in which inflation medium is supplied to the inflatable balloon. Atherectomy catheters generally require a torqueable shaft within the catheter body which allows the cutting head to be rotatably aligned with the atheroma after the catheter has been placed within the vascular system. Such torqueable shafts are typically formed from braided metal or other opaque materials which prevent viewing of the interior of the catheter. Thus, inflation supply tubes which are interior to the shaft are not visible, preventing examination for air bubbles. Air bubbles within the inflation medium are, of course, unacceptable as they could lead to air emboli should the inflatable balloon burst. Additionally, the atherectomy catheters have suffered from problems in the sealing and joining of the inflation medium supply tube(s). As most atherectomy devices employ inflatable balloons which are separate from the tube or other member supplying inflation medium, a potential failure point is found at the junction between the inflation medium supply tube and the balloon.

Another problem with atherectomy catheters (and other catheters having inflatable balloons) results from the need to purge air from the balloon as it is being inflated with incompressible inflation medium. Heretofore, balloon angioplasty devices have usually employed a small orifice permanently formed in the distal tip of the balloon to allow air bleeding during the priming of the system with inflation medium. The orifice allows air to bleed, while substantially preventing passage of the more viscous liquid inflation medium. A second approach with balloon angioplasty devices has been the incorporation of a metal vent tube extending from the balloon to a vent tube port at the proximal end of the catheter. Once the balloon is filled with contrast medium, the vent tube is removed before performing the angioplasty. This design, however, is somewhat complex and cumbersome and requires a larger diameter catheter shaft to accomodate the vent tube.

For the above reasons, it would be desirable to provide atherectomy catheters where the entire balloon inflation system within the device is visible to the user while the device is being primed with inflation medium. Such visibility will allow the user to check for air bubbles which would endanger the patient should the inflatable balloon on the device burst during use. Additionally, it would be desirable to provide atherectomy devices having improved seal means between the inflatable balloon and the tube or lumen supplying inflation medium through the length of the catheter. It would be particularly desirable if the balloon could be formed integrally (without joints) with the inflation medium supply tube or lumen. Finally, it would be desirable to provide such atherectomy catheters having improved vent means to allow purging of air during priming of the catheter with inflation medium.

### Description of the Background Art

U.S. Patent Nos. 4,323,071 and 4,411,055 disclose balloon-tipped angioplasty catheters formed from inner and outer coaxial tube members. The outer coaxial tube defines the inflation balloon at its distal end and serves to carry inflation medium along the length of the catheter. U.S. Patent Nos. 4,627,436 and 4,685,458 describe atherectomy devices which utilizes a fixed blade to sever atheroma. A lumen within the device carries inflation medium to a balloon located opposite the blade at the distal end of the device. U.S. Patent No. 4,669,469 describes a single lumen atherectomy device where both a cutter cable and inflation medium pass through the lumen. The lumen is connected to the interior of an inflation balloon and the cutter cable is sealed by an O-ring. EP-A-0,163,502 discloses an atherectomy device having a cutter housing connected via a conical member to a flexible guide member which is covered by a first flexible tube. An inflatable balloon is secured at its two ends to the cutter housing by straps, with a lumen connected to the balloon for inflating the same. The lumen is held to the flexible guide member by another tube. American Edwards Laboratories, Santa Ana, California, sells a balloon-tipped catheter having discrete inflation and vent lumens extending from the entire catheter length, as illustrated in sales sheet 132-8/86-LIS.

### SUMMARY OF THE INVENTION

The present invention provides an improved atherectomy device.

In a first aspect, the present invention provides an atherectomy catheter comprising a flexible tube defining a first lumen with a proximal end and a distal end, a flexible opaque torque member disposed within said first lumen, said torque member having an open interior, and a housing disposed within the distal end of the first lumen and joined to the torque member, said housing having means for severing atheroma, wherein said catheter further includes a second lumen formed by said flexible tube, and said second lumen is enlarged at its distal end to form a balloon adjacent said housing, the flexible tube being substantially transparent to allow a user to check visually for air bubbles in said second lumen and said balloon. This construction has particular advantages since there are no seals required between the inflation balloon and the inflation medium supply lumen. Moreover, the inflation balloon is firmly attached to the cutter housing which will normally be disposed in the second lumen with the torque tube. Thus, the inflation balloon will remain properly aligned with the cutter housing at all times.

In a second aspect, the present invention provides a method for forming an atherectomy catheter of the present invention, the method comprising constricting said first lumen of said flexible tube about said flexible torque member and said housing, and expanding a distal region of the second lumen proximate the housing to form said balloon.

In the present invention, novel vent systems can be provided for each of the catheter constructions described above. A non-removable, flexible vent tube may be inserted through the distal tip of the inflatable balloon. The vent tube, which may conveniently be fused silica, is preferably placed through an open end of the balloon during fabrication, and the balloon constricted about the vent tube by heat shrinking or by bonding with flexible adhesive. Alternatively, a rigid vent tube may be removably inserted through a distal tip of the balloon. In that case, the tube is passed through an elastomeric sleeve and a resilient strap holds the vent tube in place prior to use. The resilient strap will automatically seal the opening which is left when the vent tube is removed from the elastomeric sleeve after the catheter is primed with inflation medium. These systems are advantageous over vent systems employing a vent tube or lumen running the length of the catheter because they are less awkward to use, vent more rapidly, and do not require an increased tube diameter to accommodate the vent. They are advantageous over orifice vents because they vent more rapidly and are more reliable.

Fig. 1 is a perspective view of a catheter.

Fig. 2 is an elevational view of the distal end of the catheter of Fig. 2, shown in section.

Fig. 3 is a cross-sectional view taken along line 3-3 in Fig. 2.

Fig. 4 is a perspective view of an embodiment of the catheter of the present invention.

Fig. 5 is an elevational view of the distal end of the catheter of Fig. 4, shown in section.

Fig. 6 is a cross-sectional view taken along line 6-6 of Fig. 5.

Fig. 6A is similar to Fig. 6, but illustrates a third lumen in the catheter.

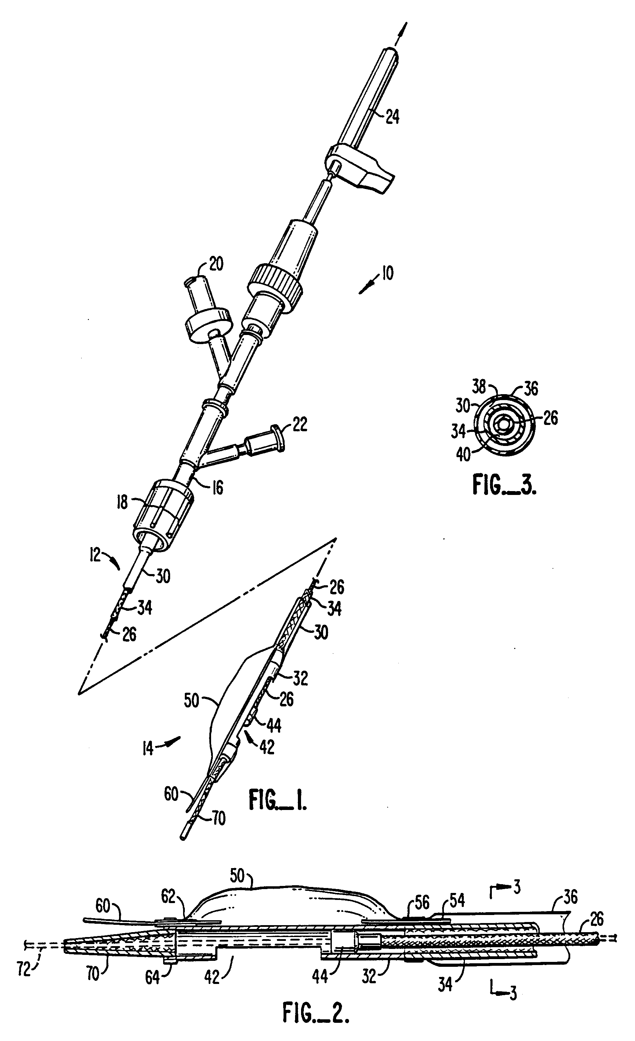

The catheter of Figs. 1-3 is not within the claims but a description is given to assist in understanding the operation of the embodiments to be described later.

Referring to Figs. 1-3, an atherectomy catheter 10 includes a proximal end 12 and a distal end 14. A manifold connector 16 at the proximal end 12 includes a conventional rotatable fitting 18 for joining the manifold connector 16 to a flexible catheter body 30. A balloon port 20 and flush port 22 are provided and connected to the catheter body 30, as described in greater detail hereinafter. A driver connection 24 is attached to cutter torque cable 26, and is able to rotate and axially translate the cable, typically using a motor drive unit (not illustrated). The design and construction of the manifold connector 16 is conventional and need not be described further. The description hereinbelow with reference to Figs. 1-3 is made for a better understanding of the concept of the present invention.

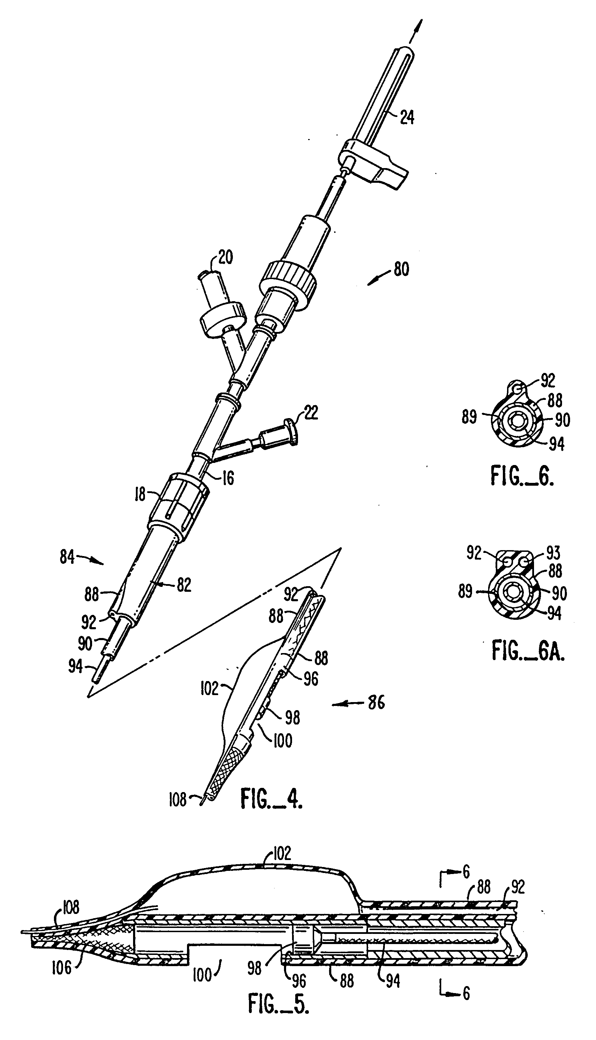

The catheter body 30 is that portion of the atherectomy catheter 10 which extends from the manifold connector 16 to a cutter housing 32 at the distal end. Referring to Fig. 3 in particular, the catheter body 30 includes a flexible torque member 34 and an outer sheath member 36 in addition to the cutter torque cable 26. The flexible torque member 34 will be fixedly attached to the manifold connector 16 so that rotation of the manifold connector will result in rotation of the catheter body along its entire length, resulting in rotation of the cutter housing 32. The flexible torque member 34 will normally be a braided metal cable, typically a stainless steel braided cable. It is important that the flexible torque member be highly flexible, yet capable of transmitting torque along its entire length with a minimal loss of transmission efficiency. The diameter of the flexible torque member 34 will vary depending on the intended application of the catheter 10, generally being in the range from about 1 mm to 4mm, usually being in the range from about 2 to 4 mm for peripheral arteries and in the range from about 1 to 2 mm for coronary arteries.

The outer sheath 36 will be transparent along its entire length, conveniently be fabricated from a thermoplastic, such as nylon, polyurethane, polyethylene terephthalate (PET), polyvinyl chloride (PVC), heat-shrinkable polyethylenes, and the like. Typically, the outer sheath 36 will have a relatively thin wall, usually having a thickness in the range from about 0.05 mm to 0.3 mm, more usually having a thickness in the range from about 0.07 mm to 0.15 mm. The diameter of the outer sheath 36 will be slightly greater than the outside diameter of flexible torque member 34, thus defining a first annular lumen 38. The width of the lumen 38 will usually be in the range from about 0.05 mm to 0.3 mm, more usually being in the range from 0.1 mm to 0.2 mm. When using a heat-shrinkable material, the diameter can be controlled precisely by constricting the sheath material about a mandrel having a desired diameter.

The cutter torque cable 26 extends through the lumen of flexible torque member 34, having a diameter in the range from about 0.4 mm to 1.5 mm, usually being in the range from about 0.5 mm to 1.0 mm. Conveniently, the cutter torque cable 26 may be formed from multi-stranded stainless steel wire. If it is desired to pass a steerable guidewire through the center, the cable 26 should be formed into a tube, typically a braided tube, such as a stainless steel braid, coated with a plastic, such as a urethane.

The first annular lumen 38 is formed between the outer sheath 36 and the outside flexible torque member 34, while a second annular lumen 40 is formed between the inside wall of flexible torque member 34 and the outside of the cutter torque cable 26. The first annular lumen 38 is connected with the balloon inflation port 20 on manifold housing 16, while the second annular lumen 40 is connected to the flush port 22. During use of the atherectomy catheter 10, the balloon inflation port 20 will be connected to a suitable source of balloon inflation medium, while the flush port 22 will be connected to a suitable flushing solution.

Cutter housing 32 is generally a hollow cylindrical structure which is fixedly attached to the distal end of flexible torque member 34, thus forming an extension thereof. The cutter housing will usually be a rigid structure, typically formed from stainless steel or other surgically acceptable metals, but may also be a flexible structure. An elongate aperture 42 is formed on a one side of the cutter housing 32, and a cup-shaped cutting blade 44 is rotatably mounted within the interior thereof. The length of the cutter housing is not critical, typically being in the range from about 10 mm to 50 mm, usually being in the range from 12 mm to 40 mm. The elongate aperture 42 will typically have a length in the range from about 5 mm to 45 mm and a width in the range from about 1 mm to 4 mm. The cutting blade 44 is attached to the distal end of cutter torque cable 26, so that the blade 44 may be rotated and axially translated by manipulation of the driver connection 24. Conveniently, motorized means for rotating and translating the cutter blade may be provided.

An inflatable balloon 50 is secured to the cutter housing 32 generally on the side opposite to the aperture 42. The inflatable balloon 50 will be formed from transparent materials, typically transparent thermoplastics, such as polyurethane-based copolymers and cross-linked polymers. Typically, the balloon will have a width, when fully inflated, of approximately 1 mm to 6 mm, more usually about 2 mm to 4 mm.

The balloon 50 must be fluidly connected to the interior lumen 38 which carries the inflation medium. Conveniently, a connector tube 54 will be provided between the annular lumen 38 and the interior of balloon 50. The distal end of the sheath 36 may then be sealed to the catheter 10 and about the tube 54, typically by heat shrinking or shrinkage. The resulting constriction 56 will provide a fluid tight seal, preventing leakage of the inflation medium. Moreover, because both the balloon 50 and sheath 36 are formed from a transparent material, the user of the catheter may easily check for bubbles which might remain in the catheter after priming. Optionally, a sealing sleeve or strap (not illustrated) may be provided around the constriction 56 in order to further assure a leak-tight seal. Alternatively, the connector tube 54 could be eliminated and the balloon tube 50 would be bonded directly to lumen 38 of sheath 36.

In order to vent air from the balloon 50, as it is being primed with inflation medium, a vent tube 60 is provided. The vent tube 60 may be formed from any surgically acceptable material, typically being stainless steel and will have a length in the range from about 10 mm to 40 mm and an inner diameter in the range from about 0.05 mm to 0.8 mm. The vent tube 60 is inserted through the distal end of balloon 50, passing through an elastomeric, typically latex or silicone, seal sleeve 62. A resilient strap 64, typically formed from nylon line, is provided about the balloon 50 at the point of entry of the vent tube 60. The strap 64 not only serves to hold the distal end of the balloon 50 in place, but also acts to seal the opening left after removing the vent tube 60. The latex sleeve 62 further assures sealing by expanding to fill the opening.

A flexible open-ended tip 70 is attached to the distal end of cutter housing 32, forming a continuous interior volume therewith. The interior volume of the tip is capable of receiving and retaining atheroma material which is severed by blade 44 as it is brought forward in the housing 32. The flexible tip 70 also facilitates positioning the catheter 10 over a conventional guidewire 72 in the vascular system. The flexible tip 70 is conveniently formed from a braided material typically braided stainless steel, and is attached to the cutter housing 32 by conventional means.

Referring now to Figs. 4-6, an alternative embodiment 80 of the catheter of the present invention will be described. The catheter 80 includes a manifold housing 16 which is substantially the same as manifold housing 16 in Fig. 1. Corresponding reference numerals refer to the same elements in both Figs. 1 and 4.

Catheter body 82 extends from proximal end 84 to distal end 86 of the atherectomy catheter 80. The catheter body 82 includes a multiple lumen flexible tube 88 including at least a first lumen 89 and a second lumen 92 (with a third lumen 93 illustrated in Fig. 6A). A flexible torque member 90 is disposed in a first lumen 89, while the second lumen 92 defines an inflation conduit and inflatable balloon 102, as will be described in more detail hereinafter. Cutter torque cable 94 passes through the interior of flexible torque member 90, and the construction of both the flexible torque member 90 and cutter torque cable 94 are substantially similar to the constructions described for the corresponding elements 34 and 26 in Figs. 1-3.

The flexible tube 88 will be composed of a translucent thermoplastic material, such as a heat-shrinkable polyethylene. The initial size of the two lumens 89 and 92 is not critical so long as they may be manipulated to assume the desired final dimensions, as described below. Initially, the first lumen 89 will be expanded using hot air and internal pressure. The flexible torque member 90 may then be inserted into the expanded lumen 89, and the lumen further heated (without internal pressure) in order to constrict the tube 88 about the flexible torque member. Conveniently, a space maintainer (not illustrated), such as a teflon rod, may be inserted in the second lumen while the flexible torque member 90 is being inserted into the first lumen 89. Conveniently, the teflon rod will have the desired diameter for the final inflation lumen so that such diameter is simultaneously achieved, while the flexible torque member 90 is being inserted. The diameter of the first lumen will depend on the outer diameter of the flexible torque member 90, typically being between about 1 mm and 4 mm, more typically being between about 1.5 mm and 3 mm. The inflation lumen 92 will have a diameter in the range from about 0.3 mm to 1.0 mm, more usually being in the range from about 0.4 mm to 0.7 mm. Optional third lumen 93 will typically be utilized for receiving a steerable guidewire (not illustrated) to allow positioning of the catheter in a conventional manner. Additional lumens (not illustrated) may be provided in the flexible tube 88 as desired for other purposes.

The distal end 86 of the catheter 80 includes a cutter housing 96 which is fixedly attached to the distal end of the flexible torque member 90. A cutter member 98 is attached to the distal end of cutter torque cable 94, and is able to be rotated and axially translated past aperture 100 formed in the cutter housing 96. The construction of the cutter housing 96 and cutter member 98 are substantially the same as described previously in Figs. 1-3.

Balloon 102 is formed by expanding the second lumen 92 adjacent to the cutter housing 96. Conveniently, expansion is achieved using hot air at an elevated pressure, and the final dimensions may be achieved by inserting an appropriate spacer block and reheating the material to provide shrinkage. The dimensions of the balloon 102 will be generally the same as described for balloon 50 hereinbefore.

A flexible tip 106 is attached to the distal end of cutter housing 96, forming a continuous interior volume therewith in a manner similar to tip 70 in Figs. 1-3. The distal end of flexible tube 88 is constricted about the flexible tip 106 by heating, and a balloon vent is provided by inserting a flexible tube 108, typically a fused silica tube, through the lumen 92 prior to constriction. Alternatively, the tube 108 can be bonded to the flexible tube 88 with a flexible adhesive. After constriction, the flexible tip 106, flexible vent tube 108 and constricted tubing 88 form a compliant tip for the catheter which facilitates positioning the catheter as described above. The flexible vent tube 108 will typically have an inner diameter in the range from about 0.05 mm to 0.3 mm.

## Claims

1. An atherectomy catheter comprising:
a flexible tube (82) defining a first lumen (89) with a proximal end (84) and a distal end (86);
a flexible opaque torque member (90) disposed within said first lumen (89), said torque member having an open interior; and characterised by:
a housing (96) disposed within the distal end of the first lumen and joined to the torque member, said housing having means (98) for severing atheroma;
wherein said catheter further includes a second lumen (92) formed by said flexible tube, and said second lumen is enlarged at its distal end to form a balloon (102) adjacent said housing; the flexible tube being substantially transparent to allow a user to check visually for air bubbles in said second lumen (92 and said balloon (102).

2. An atherectomy catheter as in claim 1, wherein the flexible torque member (90) is a braided tube having a diameter from about 1 mm to 4 mm.

3. An atherectomy catheter as in claim 1, wherein the flexible torque member (90) is a braided tube having a diameter in the range from about 0.4 mm to 1.5 mm.

4. An atherectomy catheter as in any preceding claim, wherein said housing (96) includes an axially elongated aperture (100) and said severing means comprises a rotatable blade (98) mounted within the housing and a flexible cable (94) attached to the blade, said cable being capable of rotating and axially translating the blade past the elongated aperture.

5. An atherectomy catheter as in claim 4, wherein the balloon (102) is disposed on the side of the housing (96) opposite the aperture (100).

6. An atherectomy catheter as in any preceding claim, wherein the flexible tube (82) includes at least a third lumen (93) capable of receiving a steerable guidewire (70).

7. An atherectomy catheter as in claim 1, wherein the flexible tube (82) is a flexible plastic tube having a diameter in the range from about 1 mm to 4 mm.

8. An atherectomy catheter as in any preceding claim, further including a flexible vent tube (108) disposed through a distal end of the second lumen (92) and extending into the interior of the balloon (102), whereby air within the balloon and the second lumen may be bled during inflation of the balloon with an incompressible fluid.

9. An atherectomy catheter as in claim 8, wherein the vent tube (108) is fused silica.

10. An atherectomy catheter as in claim 8 or claim 9, wherein the inner diameter of the vent tube (108) is in the range from about 0.05 mm to 0.3 mm.

11. A method for forming an atherectomy catheter of any preceding claim, said method comprising:
constricting said first lumen (89) of said flexible tube (82) about said flexible torque member (90) and said housing (96); and
expanding a distal region of the second lumen (92) proximate the housing to form said balloon (102).

12. A method as in claim 11, wherein the flexible tube (82) is composed of a thermoplastic and the first lumen is constructed by heating.

13. A method as in claim 11 or claim 12, wherein the distal region of the second lumen is expanded by heating and applying internal pressure to the interior thereof.

14. A method of any of claims 11 to 13, wherein the first lumen (89) is shrunk tightly about the flexible torque member (90).

15. A method of any of claims 11 to 14, wherein said first lumen (89) is constricted about said torque member (90) by the steps of:
a) expanding the first lumen (89) by using hot air and internal pressure;
b) inserting the torque member (90) into the expanded first lumen; and
c) heating without internal pressure the first lumen to constrict the same onto the torque member.

16. A method of any of claims 11 to 15, wherein the distal end of the balloon (102) is heat sealed about a vent tube (108).

17. A method of any of claims 11 to 15, wherein a vent tube (108) is bonded in the distal end of the balloon (102) with a flexible adhesive.

## Patentansprüche

1. Atherektomie-Katheter umfassend:
ein flexibles Rohr (82), das ein erstes Lumen (89) definiert und ein proximales Ende (84) und ein distales Ende (86) besitzt;
ein flexibles, undurchsichtiges Torsionselement (90), das im ersten Lumen (89) angeordnet ist, wobei das Torsionselement einen offenen Innenraum besitzt; gekennzeichnet durch:
ein Gehäuse (96), das innerhalb des distalen Endes des ersten Lumens angeordnet und mit dem Torsionselement verbunden ist, wobei das Gehäuse ein Mittel (98) zum Abtrennen von Atheromen aufweist;
worin der Katheter weiters ein vom flexiblen Rohr gebildetes zweites Lumen (92) umfaßt und das zweite Lumen an seinem distalen Ende vergrößert ist, um einen Ballon (102) angrenzend zum Gehäuse zu bilden; wobei das flexible Rohr im wesentlichen durchsichtig ist, um dem Benutzer die visuelle Kontrolle hinsichtlich Luftblasen im zweiten Lumen (92) und im Ballon (102) zu ermöglichen.

2. Atherektomie-Katheter nach Anspruch 1, worin das flexible Torsionselement (90) ein beflochtenes Rohr mit einem Durchmesser von etwa 1 bis 4 mm ist.

3. Atherektomie-Katheter nach Anspruch 1, worin das flexible Torsionselement (90) ein beflochtenes Rohr mit einem Durchmesser im Bereich von etwa 0,4 bis 1,5 mm ist.

4. Atherektomie-Katheter nach einem der vorhergehenden Ansprüche, worin das Gehäuse (96) eine axial verlaufende Öffnung (100) umfaßt und das Abtrennmittel eine im Gehäuse montierte, drehbare Klinge (94) und ein an der Klinge befestigtes, flexibles Kabel (94) umfaßt, wobei mit dem Kabel die Klinge gedreht und axial an der länglichen Öffnung vorbeigeschoben werden kann.

5. Atherektomie-Katheter nach Anspruch 4, worin der Ballon (102) an der der Öffnung (100) gegenüberliegenden Seite des Gehäuses (96) angeordnet ist.

6. Atherektomie-Katheter nach einem der vorhergehenden Ansprüche, worin das flexible Rohr (82) zumindest ein drittes Lumen (93) umfaßt, das einen steuerbaren Führungsdraht (70) aufnehmen kann.

7. Atherektomie-Katheter nach Anspruch 1, worin das flexible Rohr (82) ein flexibles Kunststoffrohr mit einem Durchmesser im Bereich von etwa 1 bis 4 mm ist.

8. Atherektomie-Katheter nach einem der vorhergehenden Ansprüche, weiters umfassend ein flexibles Entlüftungsrohr (108), das durch das distale Ende des zweiten Lumens (92) hindurch verläuft und sich in das Innere des Ballons (102) erstreckt, wodurch Luft innerhalb des Ballons und des zweiten Lumens während des Aufblasens des Ballons mit einer inkompressiblen Flüssigkeit abgeleitet werden kann.

9. Atherektomie-Katheter nach Anspruch 8, worin das Entlüftungsrohr (108) aus Quarzglas besteht.

10. Atherektomie-Katheter nach Anspruch 8 oder 9, worin der Innendurchmesser des Entlüfungsrohrs (108) im Bereich von etwa 0,05 bis 0,3 mm liegt.

11. Verfahren zur Bildung eines Atherektomie-Katheters nach einem der vorhergehenden Ansprüche, welches Verfahren umfaßt:
das Einengen des ersten Lumens (89) des flexiblen Rohrs (82) um das flexible Torsionselement (90) und das Gehäuse (96) herum; und
das Ausdehnen eines distalen Bereichs des zweiten Lumens (92) angrenzend zum Gehäuse zur Bildung des Ballons (102).

12. Verfahren nach Anspruch 11, worin das flexible Rohr (82) aus einem thermoplastischen Kunststoff besteht und das erste Lumen durch Erhitzen gebildet wird.

13. Verfahren nach Anspruch 11 oder 12, worin der distale Bereich des zweiten Lumens durch Erhitzen und Anlegen eines Innendrucks an dessen Innenraum ausgedehnt wird.

14. Verfahren nach einem der Ansprüche 11 bis 13, worin das erste Lumen (89) eng um das flexible Torsionselement (90) herum aufgeschrumpft wird.

15. Verfahren nach einem der Ansprüche 11 bis 14, worin das erste Lumen (89) durch die folgenden Schritte um das Torsionselement (90) herum eingeengt wird:
a) Ausdehnen des ersten Lumens (89) mittels Heißluft und Innendruck;
b) Einsetzen des Torsionselements (90) in das ausgedehnte erste Lumen; und
c) Erhitzen des ersten Lumens ohne Innendruck, um dieses um das Torsionselement herum einzuengen.

16. Verfahren nach einem der Ansprüche 11 bis 15, worin das distale Ende des Ballons (102) um ein Entlüftungsrohr (108) herum heißgesiegelt wird.

17. Verfahren nach einem der Ansprüche 11 bis 15, worin ein Entlüftungsrohr (108) im distalen Ende des Ballons (102) mit einem flexiblen Klebstoff befestigt wird.

## Revendications

1. Cathéter d'athérectomie comprenant :
un tube flexible (82) définissant une première lumière (89) avec une extrémité proximale (84) et une extrémité distale (86) ;
un membre de torsion opaque flexible (90) disposé dans ladite première lumière (89), ledit membre de torsion ayant un intérieur ouvert ; et caractérisé par :
une enceinte (96) disposée dans l'extrémité distale de la première lumière et jointe au membre de torsion, ladite enceinte ayant un moyen (98) pour trancher l'athérome ;
où ledit cathéter inclut de plus une seconde lumière (92) formée par ledit tube flexible et ladite seconde lumière est élargie à son extrémité distale pour former un ballon (102) adjacent à ladite enceinte ; le tube flexible étant substantiellement transparent pour permettre à un utilisateur de vérifier visuellement les bulles d'air dans ladite seconde lumière (92) et ledit ballon (102).

2. Cathéter d'athérectomie selon la revendication 1, dans lequel le membre de torsion flexible (90) est un tube tressé ayant un diamètre d'environ 1 mm à 4 mm.

3. Cathéter d'athérectomie selon la revendication 1 dans lequel le membre de torsion flexible (90) est un tube tressé ayant un diamètre dans l'intervalle d'environ 0,4 mm à 1,5 mm.

4. Cathéter d'athérectomie selon l'une quelconque des revendications précédentes, dans lequel ladite enceinte (96) inclut une ouverture allongée axialement (100) et ledit moyen de tranchage comprend une lame rotative (98) montée dans l'enceinte et un câble flexible (94) attaché à la lame, ledit câble étant capable de faire subir une rotation et une translation axiale à la lame au-delà de l'ouverture allongée.

5. Cathéter d'athérectomie selon la revendication 4, dans lequel le ballon (102) est disposé sur le côté de l'enceinte (96) à l'opposé de l'ouverture (100).

6. Cathéter d'athérectomie selon l'une quelconque des revendications précédentes, dans lequel le tube flexible (82) inclut au moins une troisième lumière (93) capable de recevoir un fil guide qu'on peut diriger (70).

7. Cathéter d'athérectomie selon la revendication 1 dans lequel le tube flexible (82) est un tube plastique flexible ayant un diamètre dans l'intervalle d'environ 1 mm à 4 mm.

8. Cathéter d'athérectomie selon l'une quelconque des revendications précédentes, incluant de plus un tube d'évent flexible (108) disposé à travers l'extrémité distale de la seconde lumière (92) et s'étendant à l'intérieur du ballon (102), et grâce auquel de l'air dans le ballon et la seconde lumière peut être purgé durant le gonflement du ballon avec un fluide incompressible.

9. Cathéter d'athérectomie selon la revendication 8, dans lequel le tube d'évent (108) est de la silice fondue.

10. Cathéter d'athérectomie selon la revendication 8 ou la revendication 9, dans lequel le diamètre interne du tube d'évent (108) est dans l'intervalle d'environ 0,05 mm à 0,3 mm.

11. Méthode pour former un cathéter d'athérectomie selon l'une quelconque des revendications précédentes, ladite méthode comprenant :
l'étranglement d'une première lumière (89) dudit tube flexible (82) autour dudit membre de torsion flexible (90) et de ladite enceinte (96) ; et
la dilatation d'une région distale de la seconde lumière (92) à proximité de l'enceinte pour former ledit ballon (102).

12. Méthode selon la revendication 11, dans laquelle le tube flexible (82) se compose d'un thermoplastique et la première lumière est formée par chauffage.

13. Méthode selon la revendication 11 ou la revendication 12 dans laquelle la région distale de la seconde lumière est dilatée en chauffant et en appliquant une pression interne à l'intérieur de celle-ci.

14. Méthode selon l'une quelconque des revendications 11 à 13, dans laquelle la première lumière (89) est rétrécie de manière serrée autour du membre de torsion flexible (90).

15. Méthode selon l'une quelconque des revendications 11 à 14, dans laquelle ladite première lumière (89) est étranglée autour dudit membre de torsion (90) par les étapes de :
a) dilatation de la première lumière (89) en utilisant de l'air chaud et une pression interne ;
b) insertion du membre de torsion (90) dans la première lumière dilatée ; et
c) chauffage sans pression interne de la première lumière pour l'étrangler sur le membre de torsion.

16. Méthode selon l'une quelconque des revendications 11 à 15, dans laquelle l'extrémité distale du ballon (102) est jointe à la chaleur autour d'un tube d'évent (108).

17. Méthode selon l'une quelconque des revendications 11 à 15, dans laquelle un tube d'évent (108) est fixé à l'extrémité distale du ballon (102) par un adhésif flexible.
